# EUROPÄISCHE PATENTSCHRIFT

(11) **EP 1 964 532 B1**
(45) Veröffentlichungstag und Bekanntmachung des Hinweises auf die Patenterteilung: **29.12.2010**
(21) Anmeldenummer: 08002618.0
(22) Anmeldetag: 13.02.2008
(51) Int. Cl.: A61F 2/84

(54) **Vorrichtung zum Freisetzen eines selbstexpandierenden Stents in ein Körpergefäss**
Device for releasing a self-expanding stent into a body vessel
Dispositif destiné à libérer un stent autoextensible dans un vaisseau corporel

(30) Priorität: 22.02.2007 DE 102007010305
(43) Veröffentlichungstag der Anmeldung: 03.09.2008
(73) Patentinhaber: JOTEC GmbH, 72379 Hechingen (DE)
(72) Erfinder: Fischer, Heike, Dr., 40670 Meerbusch (DE); Centola, Marcos, Sao Paulo (BR)
(74) Vertreter: Laufer, Gabriele

(56) Entgegenhaltungen:
- EP-A- 1 078 611
- WO-A-2005/067819
- US-B1- 6 514 261

## Beschreibung

Die vorliegende Erfindung betrifft eine Vorrichtung zum Freisetzen eines selbstexpandierenden Stents in ein Körpergefäß, mit einem Hüllschlauch, der in einem distalen Abschnitt den Stent radial zusammengedrückt hält, einem Schiebeelement, welches in dem Hüllschlauch geführt ist, zur Stabilisierung des Stents beim Zurückziehen des Hüllschlauchs, und einem Griff mit einer Durchführung, über welche das Schiebeelement am Griff befestigt ist.

Derartige Einführsysteme und Stents sind aus dem Stand der Technik allgemein bekannt.

Mit solchen Einführsystemen werden als endovaskuläre Stents bezeichnete Gefäßstents in Blutgefäße implantiert, welche beispielsweise auf Grund von Krankheiten oder Ähnlichem verletzt, aneurysmatisch aufgeweitet oder in ihrem Lumen verschlossen sind, wodurch die Gefäße in ihrer Funktion stark beeinträchtigt sind oder die Gefahr einer Gefäßruptur besteht. Im Stand der Technik sind verschiedene implantierbare Stentvorrichtungen bekannt, die Blutgefäße, beispielsweise Arterien, nach ihrer Implantation offen halten oder Aufweitungen von dem Blutstrom abgrenzen. Solche Stents haben in der Regel einen röhrenförmigen Körper, der in das Gefäß eingeführt und an der entsprechenden Stelle fixiert wird, um das Lumen des Gefäßes aufrecht zu erhalten.

So sind im Stand der Technik beispielsweise Stentgrafts bekannt, die ein Drahtgerüst aus einem selbstexpandierenden Material aufweisen, wobei das Drahtgerüst darüber hinaus mit einem Textil- oder PTFE-Schlauch verbunden sein kann.

Zur Implantation wird der Stent oder Stentgraft radial zusammengedrückt, so dass sich seine Querschnittsfläche deutlich verringert und er einfach in das Gefäß eingeführt werden kann. Auf Grund der Federwirkung des Metallrahmens bzw. der Metallstents expandiert der Stent wieder in seine ursprüngliche Form und spannt dabei seine Mantelfläche auf, die sich innen in dem Blutgefäß verklemmt.

Zur Implantation werden die Stents radial zusammengefaltet und dann mit Hilfe von endoluminal vorgeschobenen Kathetern in das Blutgefäß eingeführt und lagerichtig in dem Gefäß positioniert. Die richtige Lage des Stents kann dabei beispielsweise über Röntgenmarker kontrolliert werden. Damit die Stents während der Positionierung im zusammengefalteten Zustand verbleiben, sind sie in einer Hülse bzw. in einem hülsenartigen Schlauch angeordnet, der den Stent radial nach innen drückt, bzw. komprimiert. Diese sog. Rückzugshülle wird nach dem Positionieren des Stents im Gefäß zurückgezogen, wobei der Stent axial von einem Anschlag-/Schiebeelement gehalten wird, das auch als Pusher bezeichnet wird. Der Pusher liegt dabei in Anlage mit dem Stent und hält diesen in seiner axialen Lage, während die auch den Pusher umgebende Rückzugshülle von dem Stent abgezogen wird, welcher sich dabei expandieren und sich in dem Blutgefäß verklemmen kann.

Bei der Freisetzung eines selbstexpandierenden Stents oder Stentgrafts muss der Arzt dabei oft einen erheblichen Kraftaufwand am Zuggriff des Hüllschlauchs und dem Griff zur Positionierung des Implantats, der mit dem Pusher verbunden ist, aufwenden.

Der Kraftaufwand ist zum einen durch Reibung zwischen der Außenwand des stark gegen seine Expansionskräfte komprimierten Stents/Stentgrafts und der Innenwand des Hüllschlauchs bedingt, andererseits aber auch durch Reibungskräfte zwischen den beweglichen Teilen des Einführsystems und Umlenkkraftkomponenten auf Grund anatomischer Zugangsgefäß- und Aortenwindungen.

Ferner muss der behandelnde Arzt beachten, dass bei Entfernen der Hülle der Stent nicht von der Stelle, an der er gezielt platziert werden soll, verschoben werden darf.

Der hohe Kraftaufwand bei der Freisetzung eines Aortenstentgrafts belastet den Arzt physisch und kann bei Übergang von der Haft- zur Gleitreibung im Einführsystem zu einem ruckartigen Freisetzen des Stents/Stentgrafts führen. In dieser Situation ist es sehr schwierig für den Arzt, die ruckartigen Freisetzungsbewegungen am Einführsystem so zu kompensieren, dass er mit dem Pusher die Position des Stentgrafts präzise einhalten kann und gleichzeitig ein Abrutschen bzw. ein dadurch ausgelöstes Verletzen des Gefäßwand vermeidet.

Aus der EP 1 117 341 A ist ein Einführsystem für implantierbare Stents bekannt, welches ein Stabilisierungselement mit einer Rohrfeder aufweist, das sich durch den Katheter zwischen dem Außenmantel und dem Innenrohr erstreckt zum Beibehalten der Position des Stents beim Zurückziehen des Außenmantels (Pusher). Ferner weist das in der EP 1 117 341 A offenbarte System ein als längliches Gehäuse ausgebildeter Griff mit einer Durchführung auf, sowie ein in dem Gehäuse geführtes bewegliches Element mit einer Klinkennabe, welche sich auf der einen Seite im Eingriff mit an der stationären Ratsche und auf der anderen Seite sich im Eingriff mit einer beweglichen Ratsche befindet. Die bewegliche Ratsche befindet sich im Eingriff mit einem Ritzel, und dieser wiederum im Eingriff mit einem Antriebsrad, welches sich am Gehäuse befindet. Sämtliche Elemente, stationäre Ratsche, bewegliche Ratsche sowie bewegliches Element sind im Gehäuse geführt. Die Vorrichtung wird über einen am Griff vorgesehenen Hebel mit der Hand betätigt.

Im Stand der Technik sind ferner Einführsysteme mit sog. Pistolengriffen bekannt, die jedoch sehr unergonomisch sind und aufgrund der anstrengenden Bedienung des Systems beim Freisetzen des Stentgrafts zu einer Krampfhaltung des Anwenders führen können.

WO-A-2005/067819 offenbart ein Einführsystem mit den technischen Merkmalen aus dem Oberbegriff aus Anspruch 1.

Vor diesem Hintergrund ist es Aufgabe der vorliegenden Erfindung, ein alternatives Einführsystem bereitzustellen, das einfach und ohne großen Kraftaufwand zu bedienen ist, und welches eine gleichmäßige, schrittweise Freisetzung von Stents/ Stentgrafts ermöglicht.

Erfindungsgemäß wird diese Aufgabe durch die technischen Merkmale aus Anspruch 1 gelöst,

Die der Erfindung zugrunde liegende Aufgabe wird dadurch vollständig gelöst.

Das Schiebeelement bzw. der Pusher ist bei der erfindungsgemäßen Vorrichtung fest mit dem Griff und der rohrförmigen Stange verbunden. Dadurch kann sowohl die axiale als auch die rotationsbezogene Lage des Stents/Stentgrafts durch den proximalen Griff kontrolliert werden.

Mit dem offenbarten Einführsystem ist es nunmehr möglich, den Schlauch langsam und schrittweise zurückzuziehen, ohne in die Gefahr zu laufen, dass sich diese Bewegung in einen ruckartigen Mechanismus verwandelt. Mit dem beweglichen Element wird ferner die Möglichkeit geschaffen, den Hüllschlauch mit niedrigem Kraftaufwnad vom freizusetzenden Stent/Stentgraft zurückzuziehen. Alternativ kann das Einführsystem am Griff und am beweglichen Element gegriffen werden, und das bewegliche Element über bzw. an der Stange entlang gleichmäßig in Richtung des Griffs bewegt werden, so dass die Rückzugshülle gleichmäßig über den Stent hinweggleitet, der wiederum von dem Schiebeelement an der gewünschten Position festgehalten wird. Dies wird dadurch bewirkt, dass das Einführsystem bspw. vom behandelnden Arzt am Griff mit einer Hand festgehalten wird und mit der anderen Hand das bewegliche Element über die Stange zum Anwender hin - d.h. in proximale Richtung - bewegt wird. Durch diese Bewegung wird der Schlauch mitgeführt und gibt den Stent, der von dem Schlauch komprimiert wird, frei. Dadurch kann dieser sich expandieren und bspw. an die Gefäßwände anlegen.

Wie gerade erläutert, wird mit "proximaler Richtung" dabei die Richtung angegeben, die zum Anwender hinführt. Bei einer Weiterbildung der erfindungsgemäßen Vorrichtung kann vorgesehen sein, dass am beweglichen Element eine Sicherung vorgesehen ist, die ein Verrutschen des beweglichen Elements in die distale Richtung - also weg vom Anwender - und damit ggf. ein Verrutschen des Stents oder sogar eine Verletzung der Gefäßwand vermieden wird.

Das bewegliche Element weist ein Betätigungsmittel auf, durch welches das bewegliche Element im Verhältnis zur Stange schrittweise bewegt werden kann.

Diese Ausführungsform hat den Vorteil, dass der Schlauch kontrolliert, d.h. Schritt für Schritt freigesetzt werden kann, unkontrollierte, überhastete Bewegungen, die ggf. ein Verrutschen des positionierten Stents bewirken können, werden dadurch vorteilhaft vermieden.

Das Betätigungsmittel weist einen Hebelgriff und zumindest ein Schubelement auf, welches über ein erstes Ende mit dem Hebelgriff derart verbunden ist, dass die durch eine Bewegung des Hebelgriffs ausgeübte Kraft auf das Schubelement übertragen wird. Das Schubelement weist darüber hinaus ein zweites Ende zum Eingriff in die Vertiefungen zwischen den Erhebungen der Stange auf, das derart ausgebildet ist, dass eine Bewegung des beweglichen Elements in distale Richtung blockiert ist.

Dieses Merkmal hat den Vorteil, dass das bewegliche Element über den mit diesem gekoppelten Hebelgriff durch eine einfache Drückbewegung bewegt werden kann. Diese Hebelbewegung wird auf das Schubelement übertragen, das mit einem seiner Enden mit dem Hebelgriff verbunden ist. Das andere Ende des Schubelements greift in eine Vertiefung der Stange ein und überträgt diese Bewegung sowie die Kraft in axiale Richtung, also entlang der Stange und auf das bewegliche Element. Durch die Bewegung des beweglichen Elements wird gleichzeitig auch der mit dem beweglichen Element kraftschlüssig gekoppelte Hüllschlauch in proximale Richtung gezogen. Dadurch wird der Hüllschlauch über den vom Schiebeelement in Position gehaltenen Stent hinweg gezogen, wodurch der Stent freigesetzt wird. Nach Entlasten des Hebelgriffs rastet das Schubelement in die nächste Einheit der Stange, wodurch das bewegliche Element wiederum in proximale Richtung verschoben wird.

Das Schubelement kann bspw. über sein erstes Ende gelenkig mit dem Hebelgriff verbunden sein.

Vorteilhafterweise kann durch wiederholtes Betätigen und Entlasten des Hebelgriffs der Stent so schrittweise an der gewünschten Position im Gefäß freigesetzt werden, ohne dass starke Kräfte aufgewendet werden müssen und ohne dass die Gefahr von ruckartigen Bewegungen entsteht, die ev. ein Verrutschen des Stents zur Folge haben könnten.

Mit der erfindungsgemäßen Vorrichtung wird die Druckkraft, die auf den übertragen und über die Hebelwirkung um ein Vielfaches verstärkt.

Diese Vorrichtung ist also u.a. insbesondere für die Einführung von längern Stents mit größerem Durchmesser, bspw., Aorten- und abdominellen Stents geeignet. Zudem können hohe Freisetzungskräfte aufgrund von sehr verschlungenen und verkalkten Zugangsgefäßen überwunden werden.

Bei der erfindungsgemäßen Vorrichtung ist dabei bevorzugt, wenn der Hebelgriff eine der Außenformen des beweglichen Elements angepasste Form aufweist.

Diese Ausführungsform hat den Vorteil, dass der Hebelgriff mit dem beweglichen Element vollständig zusammengedrückt werden kann, so dass sich der Hebelgriff in eine Aussparung des beweglichen Elements legt und dadurch die Ausschlagsposition des Hebelgriffs in eine Richtung begrenzt ist.

Es ist ferner bevorzugt, wenn eine Feder vorgesehen ist, die den Hebelgriff in eine geöffnete Position vorspannt.

Dies hat den Vorteil, dass der Hebelgriff des beweglichen Elements somit durch die Federwirkung vom Griff abklappt; durch wiederholtes Betätigen bzw. Druckausüben auf den Hebelgriff entgegen der Federkraft in Richtung bewegliches Element wird der oben beschriebene Mechanismus in Gang gesetzt. Zur Freisetzung des Stents/Stentgrafts wird also der Hebelgriff gegen die Federkraft in Richtung bewegliches Element gedrückt, wodurch das Schubelement, das mit dem Hebelgriff verbunden ist, in die regelmäßig beabstandeten Vertiefungen der Stange rastend eingreifen kann. Durch mehrmaliges Betätigen des Hebelgriffs wird das bewegliche Element dann Schritt für Schritt zurückgezogen, wodurch der Hüllschlauch mitgezogen und der Stent freigesetzt wird. Die Kraftübertragung in axiale Richtung findet somit beim Herunterdrücken des Hebelgriffs statt. Während der Aufwärtsbewegung rastet das Schubelement dann in die nächste Vertiefung ein.

Die Feder kann bspw. zwischen dem beweglichen Element und dem Hebelgriff vorgesehen sein.

Es ist ferner bevorzugt, wenn das bewegliche Element ein Rastelement aufweist, welches im Einriff mit den Vertiefungen zwischen den Erhebungen der Stange derart ausgebildet ist, dass eine Bewegung des beweglichen Elements in distaler Richtung blockiert und in proximaler Richtung freigegeben ist.

Bei einer Weiterbildung der erfindungsgemäßen Vorrichtung ist bevorzugt, wenn ferner ein Mitnehmer vorgesehen ist, welcher in der rohrförmigen Stange axial beweglich gelagert und mit dem beweglichen Element in axialer Richtung kraftschlüssig verbunden ist, und über welchen Mitnehmer der Hüllschlauch mit dem beweglichen Element gekoppelt ist.

Diese Ausführungsform hat den Vorteil, dass bei einer axialen Bewegung des beweglichen Elements auch der mit dem beweglichen Element gekoppelten Mitnehmer bewegt wird, mit welchem wiederum der Schlauch gekoppelt, bspw. verklemmt ist. Dadurch kann der Schlauch durch die Übertragung der Bewegung auf den Mitnehmer vom Stent zurückgezogen werden, wodurch dieser freigesetzt wird. Der Mitnehmer kann dabei in einer Nut der röhrenförmigen Stange laufen, wodurch die Führung dieses Elements erleichtert ist. Mitnehmer und bewegliches Element sind dabei in axialer Richtung kraftschlüssig verbunden und bilden eine Einheit. Das bewegliche Element kann jedoch unabhängig vom Mitnehmer rotiert werden und somit in eine für den Anwender günstige Position gedreht werden. Zudem ist sichergestellt, dass durch eine Rotation des beweglichen Elements die Ausrichtung eines nicht rotationssymmetrischen Stents nicht verändert wird.

In einer weiteren Ausführungsform ist bevorzugt, wenn das Einführsystem Auslöser aufweist, durch deren Betätigung das bewegliche Element in proximale Richtung frei bewegbar ist.

Diese hat den Vorteil, dass das bewegliche Element frei, in einem Zug und unter Vermeidung von durch die Stange vorgegebenen einzelnen Schritten in Richtung des Anwenders, d.h. in proximale Richtung gezogen werden kann. Dies ist insbesondere dann erwünscht, wenn der Stent sicher platziert und schon zumindest teilweise freigesetzt wurde. Zur Beschleunigung der kompletten Freisetzung können dann die Auslöser betätigt werden, die das zumindest eine Schubelement freigeben und die bewirken, dass das Schubelement nicht mehr in die Vertiefungen der Stange eingreifen kann.

In einer weiteren Ausführungsform ist bevorzugt, wenn der Abstand zwischen den regelmäßig beabstandeten Erhebungen der Stange derart bemessen ist, dass das Schubelement nach Abfedern des Hebelgriffs vom beweglichen Element jeweils in den Grund der nächsten distalen Vertiefung zwischen den Erhebungen eingreift.

Mit dieser Ausführungsform ist gewährleistet, dass ein vorsichtiges, schrittweises Bewegen des beweglichen Elements - und damit des Schlauches - ein entsprechendes Freisetzen des Stents bewirkt werden kann. Die Länge der zurückgelegten Strecke zwischen einer Erhebung und der nächsten kann dabei je nach Ausführung der Stange und gewünschter Anwendung variieren. So kann bspw. ein Hub von ca. 1 mm bis 8 mm, insbesondere von 4 mm erreicht werden. Hierbei handelt es sich jedoch um eine variable Größe, die durch Änderung des Abstandes und/oder der Breite der Erhebungen dem jeweiligen Einsatz und Zweck angepasst werden kann.

In einer weiteren Ausführungsform ist bevorzugt, wenn die Erhebungen aufweisende Stange als eine Zähne aufweisende Zahnstange ausgebildet ist.

Diese Ausführungsform hat den Vorteil, dass das Schubelement mit seinem zweiten Ende in die Zähne eingreifen kann, wobei es durch Federkraft in den Zahngrund gedrückt wird. Die Form und Größe der Zähne kann dabei variieren. Es ist jedoch bevorzugt, wenn diejenige Fläche der Zähne, auf die das Schubelement die Kraft überträgt, eine senkrechte Fläche aufweist, und die andere Fläche der zähne eine schräge Fläche aufweist. Dadurch werden das Einrasten und das "Ratschen" des Schubelements optimal unterstützt.

Geeignete Materialien, die als Baustoffe für das erfindungsgemäße Einführsystem eines Stents eingesetzt werden können, sind - hinsichtlich der Griffteile und des beweglichen Elements bzw. Mitnehmers - bspw. verstärkte und unverstärkte Thermoplaste wie Polycarbonat (PO), Polyoxymethylen (POM), Polyester (PET), Polypropylen (PP), Polyamid (PA) Polyethylen (PE), Polyvinylchlorid (PVC), oder Mischungen davon. Ferner können Teile des Einführsystems aus Stahl oder Nichteisenlegierungen (wie bspw. Titanlegierungen) gefertigt werden.

Weitere Vorteile und Merkmale ergeben sich aus der nachfolgenden Beschreibung und den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der nachstehenden Figur dargestellt und werden in Bezug auf diese im Folgenden näher beschrieben. Es zeigt:

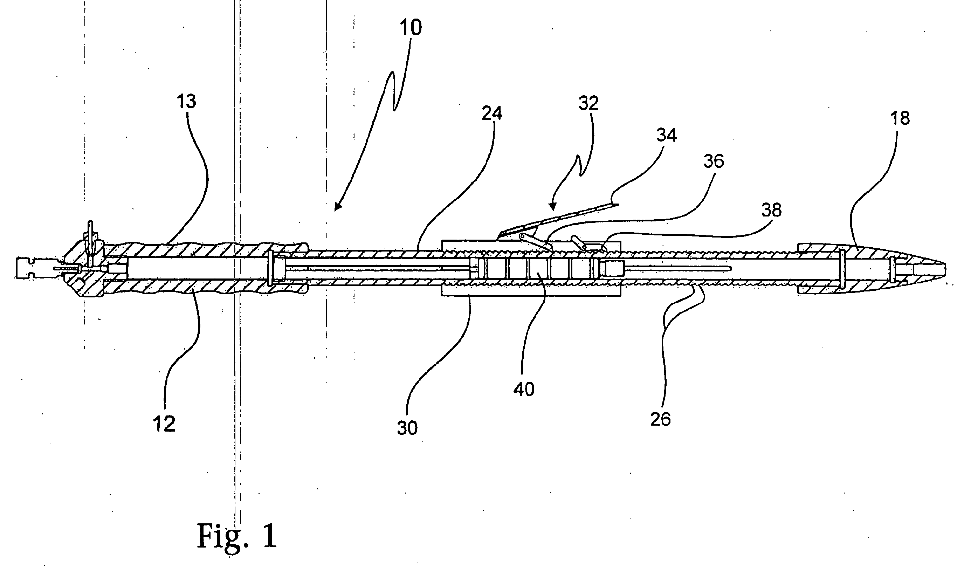

- Fig. 1: eine schematische, ausschnittsweise Darstellung eines ersten Ausfüh- rungsbeispiels der erfindungsgemäßen Vorrichtung zum Einführen eines selbstexpandierenden Stents in ein Gefäß.

Die erfindungsgemäße Vorrichtung ist in Figur 1 teilweise geschnitten und stark verkürzt sowie nicht maßstabsgetreu dargestellt.

In Fig. 1 ist ausschnittsweise eine Vorrichtung zum Einführen eines Stents gezeigt, wobei hier Elemente, die bei Stent-Einführsystemen üblich sind, aus Gründen der Übersicht weggelassen wurden. So ist in Fig. 1 der Hüllschlauch nicht gezeigt, der den Stent zur Einführung in ein Gefäß radial zusammengedrückt hält; ferner ist der geladene Stent/Stentgraft nicht gezeigt, der Drahtführungskatheter, die weiche Silikonspitze, sowie eine ev. vorhandene Fixierung für die Stentfedem am proximalen Stentende. Durch den Drahtführungskatheter kann ferner noch ein Führungsdraht geführt sein, der ebenfalls nicht dargestellt ist.

In Fig. 1 ist mit 10 insgesamt ein Griffsystem einer erfindungsgemäßen Vorrichtung bezeichnet. Dieses weist einen Griff 12 und eine Stange 24 auf. Der Griff 12 kann eine der Hand oder den Fingern eines Anwenders angepasste äußere Form aufweisen, d.h. den Fingern angepasste leichte Vertiefungen 13, die dem besseren Greifen des Griffs 12 dienen.

Ferner weist die Vorrichtung am distalen Ende eine Kappe 18 auf, die jedoch nicht in das Gefäß eingeführt wird. Die Kappe 18 ist zur Führung der Schläuche vorgesehen sowie ggf. zum Zusammenhalten der Stange, die aus zwei Halbschalen zusammengesetzt sein kann. Ferner umfasst die Vorrichtung ein Schiebeelement ("Pusher") 20, das in Fig. 1 nur angeschnitten dargestellt ist und im maßstabsgetreuen Einführsystem wesentlich länger als der Griff ist. Das Schiebeelement 20 befindet sich zur Einführung des Stents zwischen Hüllschlauch und Drahtführungskatheter in Form eines Röhrchens.

Der Griff 12 ist fest mit einer Endkappe 42 verbunden, die als Aufnahme bzw. Durchführung für das Schiebeelement, bzw. Pusher, den Drahtführungskatheter (nicht gezeigt), ggf. ein Stützrohr (nicht gezeigt), einem Spülschlauch (nicht gezeigt) und dem Auslösemechanismus einer Federfixierung (nicht gezeigt) dient. Ein Stützrohr kann innerhalb des Griffsystems eingesetzt werden, um die Stabilität des Schiebeelements (Pushers) bei axialer Belastung zu erhöhen. Das Stützrohr kann sich dazu innerhalb oder außerhalb des Pushers befinden und sollte möglichst eng an den Pusher anliegen.

Die Stange 24 ist teilweise, in Fig. 1 im distalen Bereich, mit regelmäßig beabstandeten Erhebungen 26 versehen. Die Stange 24 ist dabei direkt mit dem Griff 12 gekoppelt. Zwischen den regelmäßig beabstandeten Erhebungen sind Vertiefungen gebildet. Die Stange 24 ist in Fig. 1 als Zahnstange mit Zähnen 26 ausgebildet, die eine in die proximale Richtung weisende schräge Fläche aufweisen und damit in axialer Richtung ein asymmetrisches Zahnprofil zeigen.

Mit 30 ist in Fig. 1 das bewegliche Element bezeichnet, das über der mit dem Griff 12 verbundenen Stange 24 beweglich, also in distale Richtung verschiebbar sowie auf der Zahlstange rotierbar angeordnet ist. An dem beweglichen Element 30 ist ein Betätigungsmittel vorgesehen, das in Fig. 1 insgesamt mit 32 bezeichnet ist. Über das Betätigungsmittel kann das bewegliche Element 30 kontrolliert bewegt werden. In Fig. 1 weist das Betätigungsmittel einen Hebelgriff 34 und zumindest ein Schubelement 36 auf, das über ein erstes Ende gelenkig mit dem Hebelgriff 34 verbunden ist und das über sein zweites Ende zum Eingreifen in die Vertiefungen der Stange 24 ausgebildet ist. Durch eine in Fig. 1 nicht gezeigte Feder ist der Hebelgriff 34 vom beweglichen Element 30 vorgespannt, so dass der Hebelgriff 34 in unbelasteter Position vom beweglichen Element weggedrückt wird. Durch Drücken und Belasten des Hebelgriffs 34 in Richtung des beweglichen Elements 30 wird auch das Schubelement 36 bewegt.

Fig. 1 ist zu entnehmen, dass bei einer Betätigung des Hebelgriffs 34 diese Bewegung auf das Schubelement 36 übertragen wird, wodurch das bewegliche Teil 30 in proximale Richtung, also in Richtung des Griffes 12, geschoben wird. Während dieser Bewegung springt ein Rastelement 38 in die nächst proximal liegende Vertiefung der Stange 24. Das Rastelement 38 ist derart ausgebildet, dass es in die Vertiefung zwischen den Erhebungen der Stange 24 eingreift. Durch dieses Rastelement 38 ist die Bewegung des beweglichen Elements 30 in distale Richtung blockiert und in proximale Richtung freigegeben.

Ferner weist das Einführsystem in Fig.1 einen Mitnehmer 40 auf, der mit dem beweglichen Element 30 gekoppelt ist und in einer Nut der Stange 24 läuft, die in Fig. 1 nicht gezeigt ist. Der Hüllschlauch wird in den Mitnehmer 40 geklemmt und durch die Bewegung des beweglichen Elements 30 und dem damit gekoppelten Mitnehmer 40 in proximale Richtung bewegt, wodurch der Stent freigesetzt wird und sich expandieren kann.

Der Anwender des Einführsystems umgreift also mit einer Hand den Griff 12 und mit der anderen das Betätigungsmittel 32. Dieses kann durch mehrfaches Drücken des Hebelgriffs 34 betätigt werden. Nach jedem Loslassen klappt der Hebelgriff 34 auf Grund der Wirkung der Druckfeder immer wieder vom beweglichen Element 30 ab.

Wie bereits oben erwähnt ist zur Einführung des Stents in ein Gefäß außerdem noch ein Drahtführungskatheter mit einer atraumatischen Spitze vorgesehen, der durch das ebenfalls vom Schlauch 14 umgebene Schiebeelement ("Pusher") geführt ist. Der Pusher hält den Stent bei seiner Freisetzung in Position und wirkt der bei der Freisetzung des Stents in proximaler Richtung wirkenden Zugkraft entgegen.

Im Folgenden wird für die Fig. 1 der Mechanismus beschrieben, der bewirkt, wie sich mit jedem Druck auf den Hebelgriff 34 der Hüllschlauch schrittweise zurückzieht und den Stent freigibt.

Bei der in Fig. 1 dargestellten Ausfühmngsform kann der Hebelgriff 34 in zwei Stufen heruntergedrückt werden. Die erste Stufe entspricht dem Herabdrücken bis zum Auftreffen auf das Rastelement und dient einem schrittweisen Zurückziehen des Hüllschlauches. Drückt man den Hebelgriff weiter herunter, was der Stufe zwei entspricht, werden das Schubelement 36 und das Rastelement 38 aus den Zähnen26 der Stange 24 gehoben und das bewegliche Element kann auf der Stange 24 frei vorund zurückgeschoben werden.

Der in das Einführsystem geladene Stent wird in komprimierter Form in das gewünschte Gefäß eingeführt. Es kann bspw. ein Arretierknopf vorgesehen sein, der nach dem Einführen und vor Beginn der Stentfreisetzung zunächst entriegelt werden muss und der ein versehentliches frühzeitiges Betätigen des Hebelgriffs verhindern soll. Dazu kann der Arretierknopf verschiebbar in einer Nut des Hebelgriffs befestigt sein. Eine Relativbewegung zwischen Schlauch und Schiebeelement/Pusher ist damit nicht möglich.

Der Hebel 30 klappt - bspw. durch Entriegelung eines Arretierknopfes - dann vom beweglichen Element 30 auf Grund der Wirkung der Druckfeder ab. Das Schubelement 36 und das Rastelement 38 drücken dabei dann in die Vertiefungen der Stange 24. Die Stange 24 ist fest mit dem Griff 12 verbunden. Die Flankenwinkel des Rastelements 38 und der Stange 24 sind so gestaltet, dass eine Bewegung des Hüllschlauchs nach distal durch das Rastelement 38 blockiert wird. Das Schubelement 36 ist über ein Drehgelenk mit dem Hebelgriff 34 verbunden. Die Bewegung des beweglichen Elements überträgt sich auf die Bewegung des Hüllschlauchs, so dass dieser in proximale Richtung gezogen und der Stent freigesetzt wird.

Um den Stent/Stentgraft schneller freisetzen zu können, bzw. das Einführsystem nach der Implantation des Stents wieder verschließen zu können, kann mit der zweiten Stufe des Hebelgriffs 34 in einer anderen Ausführungsform durch Betätigung eines am beweglichen Element vorgesehenen Auslösers das bewegliche Element 30 aus dem schrittweisen Betätigungsmechanismus freigegeben und in einem Zug in proximale oder distale Richtung geführt werden.

Der Zahnabstand der Zähne auf der Stange 24 kann dabei so bemessen sein, dass das Schubelement 36 nach Loslassen des Hebelgriffs 34, wobei dieser wieder vom beweglichen Element 30 abklappt, in die nächste proximale Zahnlücke einrastet. Es versteht sich, dass die Stange nicht zwingend mit Zähnen versehen sein muss, andere Ausbildungsformen mit Erhebungen und Vertiefungen sind ebenfalls denkbar.

Mit jedem Betätigungshub des Hebelgriffs 34 wird damit der Hüllschlauch um die Strecke eines Zahnabstandes zurückgezogen.

Der Anwender der Vorrichtung kann auf diese Weise mit dem beweglichen Element 30 und der mit dem Griff 12 fest verbundenen Stange 24 komfortabel die axiale rotationsbezogene Position des Stents/Stentgrafts kontrollieren und die komplette oder teilweise Freisetzung steuern. Andererseits kann der Anwender durch Betätigen der oder des Auslösers den Hüllschlauch jederzeit in konventioneller Weise in einem Zug zurückziehen oder wieder vorschieben.

Vorteilhafterweise kann das erfindungsgemäße System außer zur Freisetzung von Stents und Stentgrafts auch bei endoluminalen Herzklappen eingesetzt werden.

## Patentansprüche

1. Vorrichtung zum Freisetzen eines selbstexpandierenden Stents in ein Körpergefäß, mit:
- einem Hüllschlauch, der in einem distalen Abschnitt den Stent radial zusammengedrückt hält,
- einem Schiebeelement, welches in dem Hüllschlauch geführt ist, zur Stabilisierung des Stents beim Zurückziehen des Hüllschlauchs,
- einem Griff (12) mit einer Durchführung, über welche das Schiebeelement am Griff (12) befestigt ist,
- eine mit dem Griff (12) fest verbundene rohrförmige Stange (24) aufweist, innerhalb derer das Schiebelement vorgsehen ist, und die zumindest teilweise mit regelmäßig beabstandeten Erhebungen (26) und zwischen den Erhebungen liegenden Vertiefungen versehen ist,
- ein bewegliches Element (30), das distal vom Griff (12) über der Stange (24) beweglich angeordnet ist, und durch dessen Bewegung in proximale Richtung der Hüllschlauch zurückziehbar ist,
wobei das bewegliche Element (30) ein Betätigungsmittel (32) aufweist, durch welches das bewegliche Element (30) im Verhältnis zur Stange (24) schrittweise bewegt werden kann, **dadurch gekennzeichnet, dass** das Betätigungsmittel (32) einen Hebelgriff (34) und zumindest ein Schubelement (36) aufweist, welches über ein erstes Ende mit dem Hebelgriff (34) verbunden ist, und welches mit seinem zweiten Ende zum Eingriff in die Vertiefungen zwischen den Erhebungen (26) der Stange (24) derart ausgebildet ist, dass eine Bewegung des beweglichen Elements (30) in distale Richtung blockiert ist.

2. Vorrichtung nach Anspruch 1, **dadurch gekennzeichnet, dass** eine Feder vorgesehen ist, die den Hebelgriff (34) in eine geöffnete Position vorspannt.

3. Vorrichtung nach einem der Ansprüche 1 oder 2, **dadurch gekennzeichnet, dass** das Betätigungsmittel (32) zumindest ein Rastelement (38) aufweist, welches im Eingriff mit den Vertiefungen zwischen den Erhebungen (26) der Stange (24) derart ausgebildet ist, dass eine Bewegung des beweglichen Elements (30) in distaler Richtung durch das Rastelement (38) blockiert und in proximaler Richtung freigegeben ist.

4. Vorrichtung nach einem der Ansprüche 1 bis 3, **dadurch gekennzeichnet, dass** ferner ein Mitnehmer (40) vorgesehen ist, welcher in der rohrförmigen Stange (24) axial beweglich gelagert und mit dem beweglichen Element (30) in axialer Richtung kraftschlüssig verbunden ist, und über welchen der Hüllschlauch mit dem beweglichen Element (30) gekoppelt ist.

5. Vorrichtung nach Anspruch 4, **dadurch gekennzeichnet, dass** die Stange (24) eine Nut zur beweglichen Aufnahme des Mitnehmers (40) in der Nut aufweist.

6. Vorrichtung nach einem der Ansprüche 1 bis 5, **dadurch gekennzeichnet, dass** zumindest ein Auslöser vorgesehen ist, welcher die freie Bewegung des beweglichen Elements (30) in proximale oder distale. Richtung ermöglicht.

7. Vorrichtung nach einem der Ansprüche 1 bis 6, **dadurch gekennzeichnet, dass** der Abstand zwischen den Erhebungen (26) der Stange (24) derart bemessen ist, dass das Schubelement (36) nach Abfedern des Hebelgriffs (34) vom beweglichen Element (30) jeweils in die nächste proximale Vertiefung eingreift.

## Claims

1. Device for releasing a self expanding stent in a body vessel, comprising:
- a tubular sheath which, in a distal section, keeps the stent radially compressed,
- a pusher element which is guided in the tubular sheath, in order to stabilize the stent when the tubular sheath is pulled back,
- a handle (12), with a passage via which the pusher element is secured on the handle (12),
- a tubular rod (24) which is fixedly connected to the handle (12) and inside which the pusher element is provided, and which is provided at least in part with regularly spaced elevations (26) and with depressions lying between the elevations,
- a movable element (30), which is arranged movably over the rod (24), distally from the handle (12), and whose movement in the proximal direction allows the tubular sheath to be pulled back,
wherein the movable element (30) comprises an actuating means (32) by which the movable element (30) can be moved step by step in relation to the rod (24), **characterized in that** the actuating means (32) comprises a lever grip (34) and at least one thrust element (36), which is connected via a first end to the lever grip (34) and which at its second end is designed to engage in the depressions between the elevations (26) of the rod (24), such that a movement of the movable element (30) in the distal direction is blocked.

2. The device of claim 1, **characterized in that** a spring is provided, which pretensions the lever grip (34) into an opened position.

3. The device of claim 1 or 2, **characterized in that** the actuating means (32) comprises at least one catch element (38) which is designed engaging with the depressions between the elevations (26) of the rod (24), such that a movement of the movable element (30) in the distal direction is blocked by the catch element (38) and is freed in the proximal direction.

4. The device of any of claims 1 to 3, **characterized in that** a carrier (40) is also provided, which is mounted axially movably in the tubular rod (24) and is connected to the movable element (30) in the axial direction with a force fit, and via which the tubular sheath is coupled to the movable element (30).

5. The device of claim 4, **characterized in that** the rod (24) has a groove for receiving the carrier (40) movably in the groove.

6. The device of any of claims 1 to 5, **characterized in that** at least one trigger is provided that permits the free movement of the movable element (30) into the proximal or distal direction.

7. The device of any of claims 1 to 6, **characterized in that** the distance between the elevations (26) of the rod (24) is such that the thrust element (36) engages, respectively, the next proximal depression after the lever grip (34) has sprung back from the movable element (30).

## Revendications

1. Dispositif destiné à libérer un stent auto extensible dans un vaisseau corporel, comportant :
- une enveloppe tubulaire qui maintient le stent comprimé radialement dans une partie distale,
- un élément poussoir qui est conduit dans l'enveloppe tubulaire pour la stabilisation du stent lors du retrait de l'enveloppe tubulaire,
- une poignée (12) dotée d'un conduit par lequel l'élément poussoir est fixé à la poignée (12),
- une tige tubulaire (24) reliée fixement à la poignée (12) à l'intérieur de laquelle l'élément poussoir est prévu et qui est dotée au moins partiellement de proéminences (26) espacées régulièrement et entre les proéminences se trouvent des creux,
- un élément mobile (30) qui est disposé sur le plan distal par rapport à la poignée (12) sur la tige (24) et peut être retiré par son mouvement dans la direction proximale de l'enveloppe tubulaire,
l'élément mobile (30) présentant un moyen d'actionnement (32) par le biais duquel l'élément mobile (30) peut être déplacé progressivement par rapport à la tige (24), **caractérisé en ce que** le moyen d'actionnement (32) présente une poignée à levier (34) et au moins un élément de poussée (36) qui est relié à la poignée à levier (34) par une première extrémité et qui, avec sa deuxième extrémité destinée à être en prise dans les creux entre les proéminences (26) de la tige (24) est conçu de telle sorte qu'un mouvement de l'élément mobile (30) soit bloqué en direction distale.

2. Dispositif selon la revendication 1, **caractérisé en ce qu'**un ressort est prévu, qui effectue une précontrainte de la poignée à levier (34) dans une position ouverte.

3. Dispositif selon l'une des revendications 1 ou 2, **caractérisé en ce que** le moyen d'actionnement (32) présente au moins un élément d'arrêt (38) qui, en prise avec les creux entre les proéminences (26) de la tige, est conçu de telle sorte qu'un mouvement de l'élément mobile (30) en direction distale soit bloqué par l'élément d'arrêt (38) et soit libéré en direction proximale.

4. Dispositif selon l'une des revendications 1 à 3, **caractérisé en ce qu'**en outre, un taquet d'entraînement (40) est prévu, qui est placé de façon mobile axialement dans la tige tubulaire (24) et est relié à l'élément mobile (30) dans la direction axiale par adhérence et par le biais duquel l'enveloppe tubulaire est couplée à l'élément mobile (30).

5. Dispositif selon la revendication 4, **caractérisé en ce que** la tige (24) présente une rainure pour la réception mobile du taquet d'entraînement (40) dans la rainure.

6. Dispositif selon l'une des revendications 1 à 5, **caractérisé en ce qu'**au moins une détente est prévue, qui permet le mouvement libre de l'élément mobile (30) dans la direction proximale ou distale.

7. Dispositif selon l'une des revendications 1 à 6, **caractérisé en ce que** l'intervalle entre les proéminences (26) de la tige (24) est mesuré de telle sorte que l'élément de poussée (36) soit en prise après la détente de la poignée à levier (34) de l'élément mobile (30) respectivement dans le creux proximal suivant.
